# EUROPÄISCHE PATENTSCHRIFT

(11) **EP 0 883 610 B1**
(45) Veröffentlichungstag und Bekanntmachung des Hinweises auf die Patenterteilung: **12.07.2000**
(21) Anmeldenummer: 96946361.1
(22) Anmeldetag: 26.02.1997
(51) Int. Cl.: C07D 257/02

(54) **VERFAHREN ZUR HERSTELLUNG VON 1,4,7,10-TETRAAZACYCLODODECAN UND DESSEN DERIVATEN**
PROCESS FOR PREPARING 1,4,7,10-TETRAAZACYCLODODECANE AND ITS DERIVATIVES
PROCEDE DE PREPARATION DE 1,4,7,10-TETRA-AZALCYCLODODECANE ET DE SES DERIVES

(30) Priorität: 26.02.1996 DE 19608307
(43) Veröffentlichungstag der Anmeldung: 16.12.1998
(73) Patentinhaber: SCHERING AKTIENGESELLSCHAFT, 13353 Berlin (DE)
(72) Erfinder: PETROV, Orlin, D-14999 Berlin (DE); PRELLE, Annette, D-12103 Berlin (DE); GRASKE, Klaus, D-14197 Berlin (DE); NICKISCH, Klaus, D-12307 Berlin (DE); RADÜCHEL, Bernd, D-13465 Berlin (DE); PLATZEK, Johannes, D-12621 Berlin (DE)
(86) Internationale Anmeldenummer: EP9700927
(87) Internationale Veröffentlichungsnummer: WO9731905

(56) Entgegenhaltungen:
- WO-A-95/31444
- WO-A-96/28420
- US-A- 3 828 023
- JOURNAL OF HETEROCYCLIC CHEMISTRY, Bd. 5, Nr. 2, April 1968, Seite 305 XP000570380 G. R. HANSEN ET AL.: "Unique Synthesis of 1,4,7,10-Tetraazacyclododecane" in der Anmeldung erwähnt
- TETRAHEDRON LETTERS, Nr. 16, 1970, Seiten 1367-70, XP000674713 S. TSUBOYAMA ET AL.: "Cyclic tetramers of optically active aziridines: 1,4,7,10- Tetrabenzyl-2,5,8,11-tetra-(R)-ethyl- 1,4,7,10-tetraazacyclododecane" in der Anmeldung erwähnt
- J. ELECTROANAL. CHEM. INTERFACIAL ELECTROCHEM., Bd. 139, Nr. 1, 1982, Seiten 207-10, XP000604005 R. KOSSAI ET AL.: "The peculiar anodic behaviour of some tetraazamacrocycles"

## Beschreibung

Die Erfindung betrifft ein Verfahren zur Herstellung von 1,4,7,10-Tetraazacyclododecan (Cyclen) und dessen Derivaten.

### Stand der Technik

1,4,7,10-Tetraazacyclododecan (Cyclen) findet breite Anwendung, sowohl als makrocyclischer Ligand, wie auch als Edukt bei der Herstellung verschiedener metallhaltiger pharmazeutisch genutzter Komplexe, wie z.B. Gadobutrol (INN), Gadobenat (INN) oder Gadoteridol (INN).

1,4,7,10-Tetraazacyclododecan wird in der Regel in einer mehrstufigen Synthese durch Cyclokondensation von zwei linearen Vorläufern hergestellt (J. Chem. Rev. 1989, 929; The Chemistry of Macrocyclic Ligand Complexes, Cambridge University Press, Cambridge, U.K. 1989).

Nachteile dieser Methode sind die große Stufenzahl, eine schlechte Gesamtausbeute sowie große Abfallmengen anorganischer Salze, die während der Synthese anfallen.

Eine prinzipiell einfacher erscheinende Methode zur Herstellung von 1,4,7,10-Tetraazacyclododecan bietet die Cyclotetramerisierung von N-substituierten Aziridinen. In der Literatur werden verschiedene Varianten dieser Reaktion beschrieben. Dabei wird zunächst aus Benzylethanolamin das entsprechende N-substituierte Aziridin hergestellt und isoliert. Das Aziridin wird anschließend in Gegenwart von Brönsted-Säuren wie z.B. p-TsOH (J. Heterocyclic Chem. 1968, 305) oder Lewis Säuren wie Trialkylaluminium (US Pat. 3,828,023) oder BF₃-Etherat (Tetrahedron Letters, 1970, 1367) in geringer Ausbeute cyclotetramerisiert. Obgleich das Verfahren nur zur Herstellung kleiner Mengen (< 5g) durchführbar ist, bildet es noch 16 Jahre nach der ersten Publikation den Stand der Technik (WO 95/31444).

Alle bisher beschriebene Cyclotetramerisierungsreaktionen erfordern die Verwendung von reinen Aziridinen, die bekanntlich starke mutagene und cancerogene Wirkung aufweisen (Roth, Giftliste, VCH Weinheim). Aus diesem Grund findet die Cyclotetramerisierug von Aziridinen, die die einfachste Methode zur Herstellung von 1,4,7,10-Tetraazacyclododecan zu sein scheint, keine praktische Anwendung in technischem Maßstab. Es besteht daher ein großes Interesse an einem technisch praktikablen, wenig umweltbelastenden und weitgehend gefahrlosen Verfahren zur Herstellung von 1,4,7,10-Tetraazacyclododecan.

Aufgabe der vorliegenden Erfindung ist es daher, ein praktikables Verfahren zur Herstellung von 1,4,7,10-Tetraazacyclododecan in technischem Maßstab bereitzustellen, das die obigen Nachteile überwindet und insbesondere die Gefährdung des Menschen durch mutagene und cancerogene Aziridinzwischenstufen vermeidet.

Diese Aufgabe wird durch das erfindungsgemäße Verfahren gelöst, wie es in den Patentansprüchen gekennzeichnet ist. Es handelt sich dabei um ein Verfahren zur Herstellung von Cyclenderivaten durch Cyclotetramerisierung von Benzylaziridinderivaten, dadurch gekennzeichnet, daß das Benzylaziridinderivat in situ erzeugt und ohne Isolierung unter Zugabe einer starken Säure zu einem Tetrabenzylcyclenderivat tetramerisiert wird und abschließend die Benzylgruppen durch Hydrierung entfernt werden.

Im Rahmen der vorliegenden Erfindung soll der Begriff Cyclenderivate sowohl 1,4,7,10-Tetraazacyclododecan, als auch solche Derivate umfassen, bei denen die Ethylenbrücken Alkylsubstituenten aufweisen. So betrifft der Begriff Cyclenderivat beispielsweise auch die Verbindungen [2S-(2α,5α,8α,11α)]-2,5,8,11-Tetramethyl-1,4,7,10-tetraazacyclododecan und [2S-(2α,5α,8α,11α)]-2,5,8,11-Tetraethyl-1,4,7,10-tetraazacyclododecan. In analoger Weise soll der Begriff Tetrabenzylcyclenderivate im Rahmen der vorliegenden Erfindung sowohl 1,4,7,10-Tetrabenzyl-1,4,7,10-tetraazacyclododecan, als solche Derivate umfassen, bei denen die Ethylenbrücken Alkylsubstituenten aufweisen. Der Begriff Benzylaziridinderivat soll im Rahmen der vorliegenden Erfindung Benzylaziridin, als auch solche Derivate umfassen, bei denen der Aziridinring Alkylsubstituenten aufweist. So betrifft der Begriff Benzylaziridinderivat beispielsweise auch die Verbindungen (S)-1-Benzyl-2-methyl-aziridin und (S)-1-Benzyl-2-ethyl-aziridin.

Die Erfindung betrifft daher ein Verfahren zur Herstellung von gegebenenfalls substituierten 1,4,7,10-Tetraazacyclododecanderivaten durch Tetramerisierung entsprechender Edukte. Bevorzugt betrifft die Erfindung die Herstellung von 1,4,7,10-Tetraazacyclododecan.

Eine bevorzugte Ausführungsform des Verfahrens geht von leicht zugänglichem Benzylethanolamin aus, das durch Erwärmen (80-150°C, bevorzugt 90-110°C) mit 1- 1,4 Equivalenten konz. Schwefelsäure in einem organischen Lösungsmittel (z.B. Toluol, Cyclohexan, Heptan u.a., Konzentration: 10-20%) und azeotrope Destillation des dabei entstehende Wassers in den entsprechenden Schwefelsäureester überführt wird. Die Reaktionszeit beträgt dabei 2 - 10 Stunden. Dieser wird mit 2-5 Equivalenten wäßriger Lauge (z.B. NaOH, KOH) erwärmt und das dabei entstehende Benzylaziridin in einem zweiten Reaktionsgefäß, das zusammen mit dem ersten ein geschlossenes System bildet, kontinuierlich azeotrop mit Wasser abdestilliert. Die so gebildete wäßrige Benzylaziridin-Emulsion kann, nach Verdünnung mit einem organischen Lösungsmittel (z.B. Ethanol, Methanol, THF), durch kontinuierliche Zugabe von mindestens 0,25 - 0,4 mol (bevorzugt 0,25-0,35 mol) einer starken Säure pro mol Benzylaziridin (d.h. equivalente Menge Säure bezogen auf das Produkt) überraschenderweise vollständig zum Tetrabenzylcyclen umgesetzt werden. Als organisches Lösungsmittel kann z.B. Ethanol, Methanol oder Tetrahydrofuran (THF) verwendet werden. Als starke Säure kann beispielweise para-Toluolsulfonsäure (p-TsOH), Methansulfonsäure (MsOH), Schwefelsäure oder BF3-Etherat verwendet werden. Das Produkt wird nach Alkalisieren (0,2-0,5 Equivalente Base, z.B. NaOH, KOH) des Reaktionsgemisches durch Kristallisation aus polaren Lösungsmitteln (z.B. THF, Ethanol, Aceton, Isopropanol, Diethylether, Ethylacetat, Furan, Dioxan, Wasser oder deren Gemischen) gewonnen und anschließend in einem organischen Lösungsmittel (Ethanol, Methanol, Isopropanol, THF) unter Zuhilfenahme eines Katalysators (Pd/C, Menge 5-20% bezogen auf das Tetrabenzylcyclenderivat, Druck: 1-20 bar) hydriert. Nach Filtrieren des Katalysators und Abdestillieren des Lösungsmittels wird das 1,4,7,10-Tetraazacyclododecan in einer Ausbeute von 45-60% der theoretischen Gesamtausbeute erhalten.

Analog zu dieser Synthese kann auch alkylsubstituiertes Benzylethanolamin eingesetzt werden, z.B. L-2-Benzylaminopropanol oder L-2-Benzylaminobutanol, um Cyclen-Derivate zu erhalten, die Verzweigungen in den Ethylenbrücken aufweisen. In einer bevorzugten Ausführungsform dieser Synthese wird (S)-1-Benzyl-2-methyl-aziridin analog zum oben beschriebenen Prozeß aus L-2-Benzylaminopropanol hergestellt und ohne Isolierung durch Tetramerisierung zum [2S-(2α,5α,8α,11α)]-2,5,8,11-Tetramethyl-1,4,7,10-tetrakis(benzyl)-1,4,7,10-tetraazacyclododecan umgesetzt, aus dem durch Hydrierung das [2S-(2α,5α,8α,11α)]-2,5,8,11-Tetramethyl-1,4,7,10-tetraazacyclododecan erhalten wird.

Das erfindungsgemäße Verfahren der Cyclotetramerisierung der Benzylaziridinderivate unterscheidet sich von den im Stand der Technik bekannten Verfahren dadurch, daß keine Isolierung des Aziridins in reiner Form erforderlich ist. Die beschriebene Vorgehensweise erlaubt somit die Prozessführung in einem geschlossenen System und damit Vermeidung der Gefährdung von Mensch und Umwelt durch das cancerogene Aziridin.

Im Gegensatz zu den im Stand der Technik bekannten Verfahren zur Cyclotetramerisierung von Benzylaziridin wird statt einer katalytischen Menge einer Säure (p-TsOH, MsOH, Schwefelsäure, BF₃-Etherat oder Trialkylaluminium) eine stöchiometrische Menge (0,25~0,35 mol bezogen auf ein Mol Benzylaziridin) verwendet. Bei Versuchen, eine Maßstabsvergrößerung der bekannten Verfahren durchzuführen, um größere Mengen 1,4,7,10-Tetraazacyclododecan auf diesem Wege herstellen zu können, wurde unter Anwendung von katalytischen Mengen p-TsOH bei der Umsetzung von der in situ hergestellten Benzyläziridin-Emulsion lediglich 12-25% der theoretischen Ausbeute erzielt. Es wurde nun gefunden, daß überraschenderweise durch kontinuierliche Zugabe von 0,25 bis 0,35 Equivalenten p-TsOH (bezogen auf das Benzylaziridin) bei 60-78 °C innerhalb von 6-9 Stunden zu der azeotrop abdestillierten Benzylaziridin-Emulsion die Ausbeute an 1,4,7,10-Tetrabenzyl-1,4,7,10-tetraazacyclododecan auf 60-65% der theoretischen Ausbeute zu verbessern ist.

Weitere Vorteile dieses Verfahrens sind die hohe Gesamtausbeute und geringere Abfallmengen (Na-Sulfat bei der Aziridinherstellung und Toluol bei der Hydrierung) im Vergleich zu bekannten Verfahren.

### Ausführungsbeispiele:

Die folgenden Beispiele sollen den Erfindungsgegenstand erläutern ohne ihn auf diese beschränken zu wollen.

### Beispiel 1

Zu einer Lösung von 95 ml Benzylethanolamin in 690 ml Toluol werden 53 ml konz. Schwefelsäure zugegeben. Die entstandene Suspension wird 2 Stunden zum Sieden erhitzt. Das dabei entstehende Wasser (14 ml) wird mit Hilfe eines Wasserabscheiders abgetrennt. Nach Abkühlung auf 20 °C wird das Reaktionsgemisch mit 1300 ml Wasser versetzt, 10 min ausgerührt und die organische Phase abgetrennt. Anschließend wird die wäßrige Phase zu einer in einem zweiten Reaktionsgefäß vorgelegten Lösung von 92,2 g NaOH in 95 ml Wasser zügig zugegeben. Das Reaktiosgemisch wird zum Sieden erhitzt. Durch eine Destillationsbrücke werden 880 g Wasser-N-Benzylaziridin-Emulsion in einem dritten Reaktiosgefäß abdestilliert. Die Emulsion wird mit 880 ml Ethanol versetzt und auf 60 °C erwärmt. Dazu wird eine Lösung von 38,0 g p-TsOH in 19 ml Wasser innerhalb 8 Stunden über eine Dosierpumpe zugegeben. Nach beendeter Zugabe wird zwei Stunden unter Rückfluß gekocht. Anschließend wird das Reaktionsgemisch mit einer Lösung von 12,0 g NaOH in 20 ml Wasser versetzt. Das ausgefallene Produkt wird filtriert und aus 600 ml 2:1 Ethanol-THF Gemisch umkristallisiert. Das so erhaltene Tetrabenzylcyclen (53 g) wird in 500 ml Isopropanol gelöst und mit 10 g Pd/C (10%) bei 80 °C und 20 bar H₂-Druck hydriert. Nach Abfiltrieren des Katalysators wird die Reaktionslösung eingeengt und das Produkt aus Toluol umkristallisiert. Es werden 15,9 g (55 % d. Th.) Cyclen als farblose Kristalle erhalten. Schm. P. 110-112 °C.

### Beispiel 2

95 ml Benzylethanolamin werden wie im Beispiel 1 beschrieben mit Schwefelsäure und anschließend mit NaOH umgesetzt. Die erhaltene wäßrige N-Benzylaziridin-Emulsion wird mit 2,6 1 Ethanol versetzt und auf 50 °C erwärmt. Dazu wird eine Lösung von 29,3 g p-TsOH in 15 ml Wasser innerhalb 8 Stunden über eine Dosierpumpe zugegeben. Nach beendeter Zugabe wird zwei Stunden unter Rückfluß gekocht. Anschließend wird das Reaktionsgemisch mit einer Lösung von 9,5 g NaOH in 20 ml Wasser versetzt. Das ausgefallene Produkt wird filtriert und aus 600 ml 2:1 Ethanol-THF Gemisch umkristallisiert. Das so erhaltene Tetrabenzylcyclen (55,7 g) wird in 500 ml Isopropanol gelöst und mit 10 g Pd/C (10%) bei 80 °C und 20 bar H₂-Druck hydriert. Nach Abfiltrieren des Katalysators wird die Reaktionslösung eingeengt und das Produkt aus Toluol umkristallisiert. Es werden 15,9 g (58 % d. Th.) Cyclen als farblose Kristalle erhalten. Schm. P. 111-113 °C.

### Beispiel 3

Wie Beispiel 1, nur die Tetramerisierung wird mit 0,33 Eq. Methansulfonsäure durchgeführt. Ausbeute 52 % Cyclen. Schm. P. 110 -112 °C.

**Tabelle 1:**

| **Vergleichende Übersicht über Bedingungen und Ausbeuten der Cyclensynthese durch Cyclotetramerisierung von Benzylaziridin** | |
|---|---|
| **Bedingungen** | **Ausbeute** |
| 0,03 Eq. p-TsOH, 95% EtOH, Rfl. (analog zu Lit.1^{∗}) | 12-25% |
| 0,33 Eq. p-TsOH, 50% EtOH, 60-80°C (Beispiel 1) | 55% |
| 0,25 Eq. p-TsOH, 75% EtOH, 50-80°C (Beispiel 2) | 58% |
| 0,33 Eq. MsOH, 50% EtOH, 70°C (Beispiel 3) | 52% |

| | |
|---|---|
| * Lit. 1: J. Heterocyclic Chem. 1968, 305. | |

## Patentansprüche

1. Verfahren zur Herstellung von gegebenenfalls alkylsubstituierten Cyclenderivaten durch Cyclotetramerisierung von gegebenenfalls alkylsubstituierten Benzylaziridinderivaten, dadurch gekennzeichnet, daß das Benzylaziridinderivat aus einem gegebenenfalls alkylsubstituierten Benzylethanolaminderivat in situ durch Umsetzung mit Schwefelsäure und anschließende Umsetzung des entsprechenden Schwefelsäureesters mit wässriger Lauge erzeugt wird,
ohne Isolierung des Benzylaziridinderivates durch Zugabe von 0,25 - 0,35 mol einer starken Säure pro Mol Benzylaziridinderivat dieses zu einem Tetrabenzylcyclenderivat tetramerisiert wird und
abschließend die Benzylgruppen durch katalytische Hydrierung entfernt werden.

2. Verfahren gemäß Anspruch 1, dadurch gekennzeichnet, daß das gegebenenfalls alkylsubstituierte Cyclenderivat 1,4,7,10-Tetraazacyclododecan ist.

3. Verfahren zur Herstellung von gegebenenfalls alkylsubstituierten Tetrabenzylcyclenderivaten durch Cyclotetramerisierung von gegebenenfalls alkylsubstituierten Benzylaziridinderivaten, dadurch gekennzeichnet, daß
das Benzylaziridinderivat aus einem Benzylethanolaminderivat in situ durch Umsetzung mit Schwefelsäure und anschließende Umsetzung des entsprechenden Schwefelsäureesters mit wässriger Lauge erzeugt wird und
ohne Isolierung des Benzylaziridinderivates durch Zugabe von 0,25 - 0,35 mol einer starken Säure pro Mol Benzylaziridinderivat dieses zu einem Tetrabenzylcyclenderivat tetramerisiert wird.

4. Verfahren gemäß Anspruch 1 oder 3, dadurch gekennzeichnet, daß das Cyclenderivat [2S-(2α,5α,8α,11α)]-2,5,8,11-Tetramethyl-1,9,7,10-tetraazacyclododecan ist.

5. Verfahren gemäß Anspruch 1 oder 3, dadurch gekennzeichnet, daß die verwendete Säure para-Toluolsulfonsäure, Methansulfonsäure oder Schwefelsäure ist.

## Claims

1. Process for preparing optionally alkyl-substituted cyclen derivatives by cyclotetramerization of optionally alkyl-substituted benzylaziridine derivatives, characterized in that the benzylaziridine derivative is generated in situ from an optionally alkyl-substituted benzylethanolamine derivative by reaction with sulphuric acid and subsequent reaction of the corresponding sulphuric acid ester with aqueous base,
the benzylaziridine derivative is tetramerized without isolation by addition of 0.25-0.35 mol of a strong acid per mole of benzylaziridine derivative to give a tetrabenzylcyclen derivative and the benzyl groups are subsequently removed by catalytic hydrogenation.

2. Process according to Claim 1, characterized in that the optionally alkyl-substituted cyclen derivative is 1,4,7,10-tetraazacyclododecane.

3. Process for preparing optionally alkyl-substituted tetrabenzylcyclen derivatives by cyclotetramerization of optionally alkyl-substituted benzylaziridine derivatives, characterized in that the benzylaziridine derivative is generated in situ from a benzylethanolamine derivative by reaction with sulphuric acid and subsequent reaction of the corresponding sulphuric acid ester with aqueous base, and the benzylaziridine derivative is tetramerized without isolation by addition of 0.25-0.35 mol of a strong acid per mole of benzylaziridine derivative to give a tetrabenzylcyclen derivative.

4. Process according to Claim 1 or 3, characterized in that the cyclen derivative is [2S-(2α,5α,8α,11α)]-2,5,8,11-tetramethyl-1,4,7,10-tetraazacyclododecane.

5. Process according to Claim 1 or 3, characterized in that the acid used is paratoluenesulphonic acid, methanesulphonic acid or sulphuric acid.

## Revendications

1. Procédé de préparation de dérivés cyclène, le cas échéant substitués par un alkyle, par cyclotétramérisation de dérivés benzylaziridine le cas échéant substitués par un alkyle, caractérisé en ce que le dérivé benzylaziridine est produit in situ à partir d'un dérivé benzyléthanolamine, le cas échéant substitué par un alkyle, par transformation avec de l'acide sulfurique et transformation ultérieure de l'ester d'acide sulfurique correspondant avec de la lessive alcaline aqueuse,
sans isolation du dérivé benzylaziridine, celui-ci est tétramérisé en un dérivé tétrabenzylcyclène par addition de 0,25 - 0,35 mole d'un acide fort par mole de dérivé benzylaziridine et finalement les groupements benzyle sont éliminés par hydrogénation catalytique.

2. Procédé suivant la revendication 1, caractérisé en ce que le dérivé cyclène, le cas échéant substitué par un alkyle, est un 1,4,7,10-tétraazacyclododécane.

3. Procédé de préparation de dérivés tétrabenzylcyclène, le cas échéant substitués par un alkyle, par cyclotétramérisation de dérivés benzylaziridine, le cas échéant substitués par un alkyle, caractérisé en ce que
le dérivé benzylaziridine est produit in situ à partir d'un dérivé benzyléthanolamine par transformation avec de l'acide sulfurique et transformation ultérieure de l'ester d'acide sulfurique correspondant avec de la lessive alcaline aqueuse et, sans isolation du dérivé benzylaziridine, celui-ci est tétramérisé en un dérivé tétrabenzylcyclène par addition de 0,25 - 0,35 mole d'un acide fort par mole de dérivé benzylaziridine.

4. Procédé suivant la revendication 1 ou 3, caractérisé en ce que le dérivé cyclène est le [2S-(2α,5α,8α,11α)]-2,5,8,11-tetraméthyl-1,4,7,10-tétraazacyclododécane.

5. Procédé suivant la revendication 1 ou 3, caractérisé en ce que l'acide utilisé est de l'acide para-toluènesulfonique, de l'acide méthanesulfonique ou de l'acide sulfurique.
